# FASCICULE DE BREVET EUROPEEN

(11) **EP 0 466 537 B1**
(45) Date de publication et mention de la délivrance du brevet: **02.02.1994**
(21) Numéro de dépôt: 91401664.7
(22) Date de dépôt: 20.06.1991
(51) Int. Cl.: A61F 5/02

(54) **Système de verrouillage en une position angulaire déterminée d'un montant monté pivotant par rapport à un support**
Verriegelungssystem für eine bezüglich einer Auflage schwenkbaren Stütze in einer festgelegten Winkelposition
System for locking a predetermined angular position of a stud pivotably mounted on a support

(30) Priorité: 13.07.1990 FR 9008975
(43) Date de publication de la demande: 15.01.1992
(73) Titulaire: ETABLISSEMENTS PROTEOR Société anonyme dite:, 21100 Dijon (FR)
(72) Inventeur: Dauny, Gérald, F-21240 Savigny-les-Beaune (FR)
(74) Mandataire: Jolly, Jean-Pierre

(56) Documents cités:
- DE-U- 8 804 683
- US-A- 2 886 031
- US-A- 4 905 678

## Description

La présente invention concerne un système de verrouillage en une position angulaire déterminée d'une pièce longitudinale, appelée montant dans la suite de la présente description, montée pivotante par rapport à un support.

Dans de nombreuses techniques, des montants sont montés pivotants autour d'un axe par rapport à un support et sont livrés ainsi aux utilisateurs, qui, en fonction des conditions d'emploi, déterminent la position angulaire précise qui doit être assignée au montant, l'amènent dans cette position et doivent ensuite le verrouiller en position.

C'est le cas, par exemple, pour des montants d'orthèses ou "attelles", qui permettent la modification de l'orientation de deux segments anatomiques l'un par rapport à l'autre (voir document US-A-4 905 678, figure 8).

Dans une telle application, le système de verrouillage doit être simple, peu encombrant et d'une grande facilité d'utilisation. Il doit en outre pouvoir s'adapter sans problème à des positions angulaires largement différentes les unes des autres et se prêter facilement à des verrouillages et des déverrouillages successifs, lorsque l'on amène par approximation le montant à sa position définitive.

C'est en vue de répondre à ces exigences qu'a été conçu le présent système de verrouillage, qui, comme on l'exposera ci-après, convient particulièrement bien au réglage en position angulaire de deux segments ou parties d'une orthèse entre elles, indépendamment de la distance qui les sépare.

A cet effet, l'invention a pour objet un système de verrouillage en une position angulaire d'un montant rectiligne monté pivotant par rapport à un support, ce système étant caractérisé en ce qu'il comprend un pontet monté coulissant par rapport au support et des moyens pour rendre ce pontet rigidement solidaire de ce support en toute position relative, le pontet comportant une saignée disposée transversalement à la direction du coulissement par rapport au support et dans laquelle est engagée une partie du montant présentant deux flancs parallèles à l'axe de rotation de ce support, ladite saignée comportant elle-même des flancs latéraux en forme de portions d'un même cylindre à section circulaire dont la concavité est tournée vers les flancs parallèles du montant, des cales en forme de demi-lune étant interposées entre les flancs de la saignée et ceux du montant, le profil de ces cales étant tel qu'elles soient en contact mutuel avec lesdits flancs.

Ainsi qu'on l'exposera ci-après plus en détail, en référence aux dessins annexés, lorsque le pontet est libre de coulisser par rapport au support, tout mouvement de rotation du montant entraîne, d'une part, un déplacement rectiligne du pontet dans sa direction de coulissement, d'autre part, un mouvement de rotation des cales entre les flancs associés du montant et de la saignée, avec lesquels elles demeurent ainsi respectivement en contact. Il est donc possible d'amener avec précision le montant dans la position angulaire désirée, tout en maintenant les cales au contact de ses flancs parallèles et au contact des flancs de la saignée. En fixant alors le pontet sur le support dans cette position, on immobilisera les cales et l'on verrouillera ainsi le montant dans la position angulaire souhaitée.

Pour rendre le pontet coulissant par rapport au support et l'immobiliser en position, divers moyens pouvant être utilisés. On pourra, par exemple, ménager dans le support des lumières oblongues dirigées dans la direction du coulissement du pontet et percer celui-ci en des positions correspondantes de trous de vis, dans lesquels pourront être engagées des vis coopérant avec des écrous.

L'invention va être décrite ci-après plus en détail, en référence aux dessins annexés, sur lesquels :
La figure 1 est une vue en perspective illustrant schématiquement une forme de réalisation du système de verrouillage conforme à l'invention ;
La figure 2 est une vue partielle illustrant l'utilisation de ce système pour verrouiller en position relative deux segments d'orthèse.

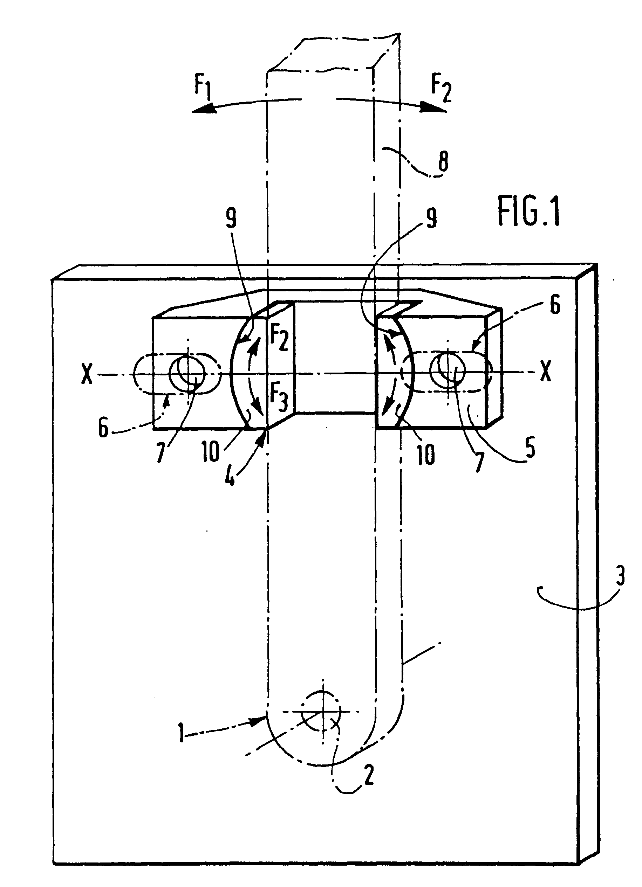

Comme on le voit sur la figure 1, un montant longitudinal 1 est monté pivotant autour d'un axe 2, par rapport à un support plan 3, et l'on souhaite pouvoir le verrouiller en diverses positions angulaires par rapport à ce support, après l'avoir fait pivoter suivant les flèches F₁ ou F₂, par rapport à la position représentée sur le dessin.

Dans ce but, conformément à l'invention, le montant 1 est engagé dans une saignée 4 d'un pontet 5, qui coiffe le montant et est susceptible de coulisser suivant la direction X-X par rapport au support 3. Afin de permettre ce coulissement et de rendre le pontet 5 rigidement solidaire du support 3 en diverses positions, deux lumières oblongues 6 sont ménagées dans le support 3 parallèlement à la direction X-X et des trous de vis 7 sont percés dans le pontet 5 en regard des lumières 6, de manière à pouvoir le fixer sur le support par un système de vis et d'écrous.

La saignée 4 du pontet est disposée transversalement à la direction X-X du coulissement de celui-ci et la partie du montant 1 engagée dans cette saignée comporte deux flancs latéraux 8, parallèles à l'axe géométrique de rotation du montant. La saignée est elle aussi délimitée latéralement par des flancs 9, en forme de portions d'un même cylindre à section circulaire, et leur concavité est tournée vers les flancs 8 du montant.

Entre les flancs 8 du montant et les flancs 9 de la saignée sont interposées, en contact mutuel avec ces flancs, des cales 10, en forme de demi-lune, qui sont pincées entre les flancs contigus, mais libres de se déplacer en rotation, par exemple suivant les flèches F₃ et F₄, par rapport aux flancs cylindriques 9, et en translation, parallèlement aux flancs 8.

On conçoit donc que, lorsque le pontet 5 est libre de coulisser par rapport au support 3, tout pivotement du montant 1 autour de son axe de rotation 2 se traduit par un coulissement du pontet 5 et par une rotation des cales 10, qui restent en contact, respectivement, avec les flancs cylindriques 9 et les flancs 8. Il est ainsi possible d'amener le montant 1 dans la position angulaire désirée par rapport au support 3, puis de le verrouiller ensuite en cette position en rendant le pontet 5 rigidement solidaire du support 3 par des vis et des écrous.

On pourrait, bien entendu, utiliser tout autre moyen de coulissement du pontet par rapport à son support et de fixation du pontet sur ce support sans sortir du cadre de l'invention.

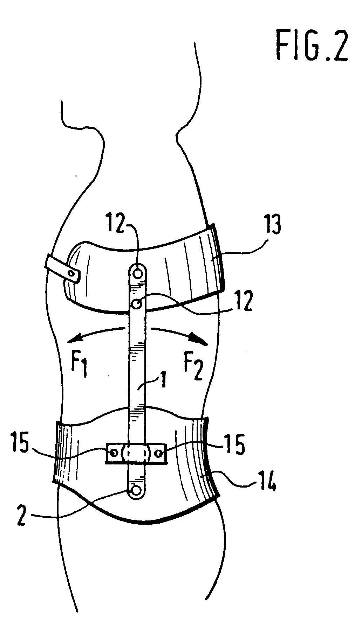

La figure 2 illustre l'application du système de verrouillage conforme à l'invention au verrouillage en position angulaire d'un segment d'orthèse par rapport à un autre.

Dans cette réalisation, le montant 1 du système conforme à l'invention est fixé à son extrémité supérieure par deux vis 12 sur un segment supérieur d'orthèse 13, tandis qu'à son autre extrémité, il est monté pivotant autour de l'axe 2 sur un segment inférieur 14 de la même orthèse.

Le pontet 5 est rendu solidaire du segment inférieur 14 par deux vis 15, engagées dans les trous de vis 7 (non représentés) du pontet et dans les lumières oblongues 6 (non représentées) ménagées dans le segment inférieur 14.

Il est donc possible de régler avec précision la position des segments 13 et 14 l'un par rapport à l'autre en ajustant la position angulaire du montant 1.

L'invention apporte donc un système de verrouillage dans une position angulaire déterminée d'un montant longitudinal monté pivotant sur un support, qui est d'une grande simplicité d'utilisation et d'un coût réduit et qui peut être utilisé avec avantage pour le réglage en position angulaire d'un segment d'orthèse par rapport à un autre.

## Revendications

1. Système de verrouillage en une position angulaire prédéterminée d'un montant rectiligne (1) monté pivotant par rapport à un support (3), ce système comprenant un pontet (5) monté coulissant par rapport au support (3) et des moyens pour rendre ce pontet (5) rigidement solidaire de ce support en toute position relative, le pontet (5) comportant une saignée (4), disposée transversalement à la direction (X-X) de coulissement par rapport au support et dans laquelle est engagée une partie du montant (1) présentant deux flancs (8) parallèles à l'axe de rotation (2) de ce support, ce système étant caractérisé en ce que ladite saignée comporte elle-même des flancs latéraux (9) en forme de portions d'un même cylindre à section circulaire dont la concavité est tournée vers les flancs parallèles (8) du montant (1), des cales (10) en forme de demi-lune étant interposées entre les flancs de la saignée et ceux du montant, le profil de ces cales étant tel qu'elles soient en contact mutuel avec lesdits flancs (8,9).

2. Système selon la revendication 1, caractérisé en ce que le support (3) comporte au moins une lumière oblongue (6) parallèle à la direction (X-X) de coulissement du pontet (5), tandis que celui-ci est percé d'au moins un trou de vis (7), disposé en regard de la lumière (6) et permettant de solidariser le pontet et le support à l'aide d'une vis et d'un écrou.

## Patentansprüche

1. System zur Verriegelung einer geraden und relativ zu einer Halterung (3) drehbar angeordneten Stütze (1) in einer Winkelposition mit einem Bügel (5), der relativ zur Halterung (3) verschoben werden kann, und mit Vorrichtungen zur starren Befestigung des Bügels (5) in jeder relativen Position auf der Halterung, wobei der Bügel (5) einen Einschnitt (4) umfaßt, der quer zur Richtung (X-X) der Verschiebung relativ zur Halterung ausgebildet ist und in den ein Teil der Stütze (1) mit zwei parallel zur Drehachse (2) der Halterung ausgebildeten Flanken (8) eingreift, wobei das System dadurch gekennzeichnet ist, daß der Einschnitt selbst seitliche Flanken (9) in Form von Abschnitten eines gleichen Zylinders von kreisrundem Querschnitt umfaßt, deren konkave Seite den parallelel Flanken (8) der Stütze (1) zugewandt ist, wobei Keile (10) in Form eines Exzenteranlaufs zwischen den Flanken des Einschnitts und denen der Stütze eingesetzt sind und wobei das Profil dieser Keile so gewählt ist, daß sie mit den Flanken (8, 9) in Eingriff gelangen können.

2. System nach Anspruch 1, dadurch gekennzeichnet, daß die Halterung (3) parallel zur Richtung (X-X) der Verschiebung des Bügels (5) mindestens ein Langloch (6) umfaßt, wobei in den Bügel mindestens ein dem Langloch (6) gegenüberliegendes Schraubloch (7) gebohrt ist, durch das der Bügel und die Halterung mittels einer Schraube und einer Mutter fest miteinander verbunden werden können.

## Claims

1. A system for locking in a predetermined angular position a rectilinear vertical member (1) mounted pivotally with respect to a support (3), this system comprising a connector bar (5) mounted slidingly with respect to the support (3) and means of firmly connecting this connector bar (5) rigidly to this support in any relative position, the connector bar (5) comprising a cut (4) arranged in transverse relation to the sliding direction (X-X) with respect to the support and in which there is engaged a part of the vertical member (1) having two flanks (8) parallel with the axis of rotation (2) of this support, this system being characterized in that said cut itself comprises lateral flanks (9) in the form of portions of one and the same cylinder of circular section, the concavity of which is turned towards the parallel flanks (8) of the vertical member (1), half-moon-shaped blocks (10) being interposed between the flanks of the cut and those of the vertical member. the profile of these blocks being such that they are in mutual contact with said flanks (8, 9).

2. A system according to claim 1, characterized in that the support (3) comprises at least one oblong slot (6) parallel with the sliding direction (X-X) of the connector bar (5), while the latter is pierced with at least one screw hole (7) arranged opposite the slot (6) and permitting firm connection of the connector bar and the support with the aid of a screw and a nut.
